Europäisches Patentamt

(19) European Patent Office

Office européen des brevets

(11) EP 0 942 260 A1

## (12) EUROPEAN PATENT APPLICATION
published in accordance with Art. 158(3) EPC

(43) Date of publication:
15.09.1999 Bulletin 1999/37

(21) Application number: 97912555.6

(22) Date of filing: 25.11.1997

(51) Int. Cl.[6]: G01B 11/06, A61B 5/14

(86) International application number:
PCT/JP97/04301

(87) International publication number:
WO 98/23916 (04.06.1998 Gazette 1998/22)

(84) Designated Contracting States:
DE GB IT NL

(30) Priority: 26.11.1996 JP 31434496
26.11.1996 JP 31434596

(71) Applicant: OMRON CORPORATION
Kyoto-shi, Kyoto 616 (JP)

(72) Inventors:
• YAMAMOTO,Katsuyuki;-Graduate School of
Engineering
Hokkaido 060 (JP)
• NIWAYAMA,Masatsugu;-Graduate School of
Engineering
Hokkaido 060 (JP)
• SHIGA, Toshikazu-Omron Corporation
Kyoto 616 (JP)
• TANABE, Kazuhisa-Omron Corporation
Kyoto 616 (JP)

(74) Representative:
Kilian, Helmut, Dr.
Wilhelms, Kilian & Partner
Patentanwälte
Eduard-Schmid-Strasse 2
81541 München (DE)

(54) **METHOD AND APPARATUS FOR MEASURING CONCENTRATION OF LIGHT ABSORBING MATERIAL IN LIVING TISSUE AND THICKNESS OF INTERCALARY TISSUE**

(57) The present invention is directed to a measuring method that permits measurement of the concentration of light absorbing material and the thickness of intercalated tissue without being affected by the thickness of intercalated tissue between the surface of an organism and the tissue to be measured. A sensor or a plurality of sensors (11-14) having different distances between the sender and receiver of light is used to measure the thickness of intercalated tissue between the surface of the organism and the organic tissue in which the light absorbing material as a test target primarily exists. Changes in measurement sensitivity with respect to the thickness of intercalated tissue at respective distances between the sender and receiver of light are examined, and based on the changes in measurement sensitivity, a relation between the thickness of intercalated tissue and a coefficient of correction for the measurement sensitivity is determined. The coefficient of correction is calculated, based on the above relation, from a known distance of the sender and receiver of light of the sensor and the thickness of intercalated tissue at a site of measurement. The measured value can be corrected using the obtained coefficient of correction, so as not to be influenced by the intercalated tissue.

FIG.1

EP 0 942 260 A1

Printed by Xerox (UK) Business Services
2.16.7/3.6

## Description

Technical Field

The present invention relates to a method of measuring the concentration of a light absorbing material such as hemoglobin within organic tissue uninvasively using near infrared rays, a method of measuring the thickness of tissue intervening between the surface of an organism and the organic tissue in which the light absorbing material as a test target primarily exists, and a measuring device thereof.

Background Art

A device for measuring oxygen in tissue using near infrared rays, for example, irradiates the surface of an organism with the near infrared rays, receives light reflected from the organic tissue, and measures oxygen in tissue based on the amount of light received. National Publication of Translation No. 4-502563, for example, describes a hemoglobin measuring instrument worn by the test subject which uses near infrared rays to measure the metabolic state of tissue of the test subject during exercise. This type of device is extremely useful for examining changes in metabolism of the brain or muscular tissue over time, and it is anticipated to be widely used in such fields as patient monitoring, emergency medicine, rehabilitation medicine, and sports medicine.

When measuring a light absorbing material, such as oxygenated hemoglobin, within muscular tissue, for example, from the surface of an organism using near infrared rays, subcutaneous adipose tissue as intercalated tissue exhibits light absorbency which is one digit smaller than that of muscular tissue because of almost no blood existing therein. Thus, the adipose tissue transmits a great amount of near infrared rays. A conventional device, however, measures oxygen in the muscular tissue and in the subcutaneous adipose tissue as a total, and does not distinguish between the amount of oxygen in the muscular tissue and that in the subcutaneous adipose tissue. This type of measurement that does not take into account effects of intercalated tissue like the adipose tissue will cause problems as the use of measurement of oxygen in tissue using near infrared rays spreads.

In other words, when measuring muscular tissue, for example, the adipose tissue between the surface of the organism and the muscular tissue hinders oxygen measurement of the muscular tissue only, as the measurement includes the adipose tissue and is affected by the thickness thereof. Therefore, measurement sensitivity will vary considerably. The same is true for the measurement of brain tissue. Cranial bones exist between the surface of the head and brain tissue, and cerebrospinal fluid exists between the cranial bones and the brain tissue. Measurement involving the intercalated tissue will create variation in measurement sensitivity.

Therefore, it is impossible to compare measurements of tested individuals, and those values are only used as relative values for measurement of the speed and pattern of change, and not evaluated as absolute values and therefore these values cannot be used for diagnosis.

Differing from the continuous light method described above, a method of measuring an absolute value indicating degree of saturation of oxygen by employing time-resolution has also been developed. However, this method also includes measurement from the surface of the organism, and the existence of intercalated tissue such as adipose tissue affects measured values considerably. This is a serious problem common to the continuous light as well as the time-resolution methods.

The present invention solves the problem in the prior art described above. An object of this invention is to provide method and device for measuring concentration of a light absorbing material within organic tissue, which allow measurement of concentration of the light absorbing material without being affected by the thickness of the intervening tissue between the surface of an organism and the tissue to be measured.

Another object of the present invention is to provide method and device for measuring the thickness of intercalated tissue of an organism, which allow measurement of the thickness of tissue intervening between the surface of the organism and the organic tissue in which the light absorbing material as a test target primarily exists.

Disclosure of the Invention

To achieve the above-described objects, the measuring method according to an aspect of the present invention is characterized in that it measures changes in light absorbency at the surface of a material using a plurality of sensors having different distances between the sender and receiver of light, and calculates the thickness of a layer of light absorbing material based on the rate of the change in light absorbency obtained.

According to another aspect of the present invention, the measuring method is characterized in that it measures changes in light absorbency within organic tissue using a single or a plurality of sensors having different distances between the sender and receiver of light, and calculates the thickness of a layer of light absorbing material based on the rate of the change in light absorbency obtained.

According to a further aspect of the present invention, the measuring method is characterized in that it measures changes in light absorbency at the surface of an organism using a single or a plurality of sensors having different distances between the sender and receiver of light, and calculates the thickness of a layer of adipose tissue within the organism based on the rate of the change in light absorbency obtained.

According to a still further aspect of the present invention, the measuring method is characterized in that it measures, using a single or a plurality of sensors having different distances between the sender and receiver of light, a linear change in the quantity of light received when the quantity of light emitted to an organism is altered linearly, determines a coefficient of proportionality in the obtained linear change from known distances of the sender and receiver of light of the one or more sensors, and calculates the thickness of a layer of light absorbing material based on the determined coefficient of proportionality.

According to another aspect of the present invention, the device for measuring the thickness of a layer of light absorbing material is characterized in that it includes a single or a plurality of sensors having different distances between the sender and receiver of light; light absorbency change measuring means for measuring, using the one or more sensors, the change in light absorbency with respect to the thickness of the layer of light absorbing material; and light absorbing material thickness calculating means for calculating the thickness of the layer of light absorbing material based on the rate of the change in light absorbency obtained.

According to a further aspect of the present invention, the device for measuring the thickness of adipose tissue of an organism includes a single or a plurality of sensors having different distances between the sender and receiver of light; light absorbency change measuring means for measuring, using the one or more sensors, the change in light absorbency at the surface of the organism; and adipose tissue thickness calculating means for calculating the thickness of adipose tissue based on the rate of the obtained change in light absorbency.

According to a still further aspect of the present invention, the device for measuring the thickness of intercalated tissue of an organism is characterized in that it includes a single or a plurality of sensors having different distances between the sender and receiver of light; quantity of received light measuring means for measuring, using the one or more sensors, a linear change in the quantity of light received when the quantity of light emitted to the organism is altered linearly; and intercalated tissue thickness calculating means for determining a coefficient of proportionality in the obtained linear change from known distances of the sender and receiver of light of the one or more sensors, and calculating, based on the determined coefficient of proportionality, the thickness of intercalated tissue between the surface of the organism and organic tissue in which the light absorbing material as a test target primarily exists.

Preferably, the sensor is characterized in that it includes a light emitting element for emitting light with a plurality of different wavelengths.

More preferably, the measuring device includes a probe provided with a plurality of mounting portions each for detachably holding a light-emitting element or a light-receiving element of the sensor, thereby differing the distance between the sender and receiver of light.

According to another aspect of the present invention, the method of measuring the concentration of a light absorbing material within organic tissue is characterized in that it measures, using a single or a plurality of sensors having different distances between the sender and receiver of light, the thickness of intercalated tissue between the surface of an organism and the organic tissue in which the light absorbing material as a test target primarily exists; examines changes in measurement sensitivity with respect to the thickness of intercalated tissue at respective distances between the sender and receiver of light; determines, based on the examined change in measurement sensitivity, a relation between the thickness of intercalated tissue and a coefficient of correction for measurement sensitivity; calculates, from the determined relation, the coefficient of correction according to known distances of the sender and receiver of light of the one or more sensors and the thickness of intercalated tissue at a site to be measured; and corrects a measured value based on the calculated coefficient of correction so as not to be influenced by the intercalated tissue.

More preferably, the measurement of the thickness of intercalated tissue is characterized in that the quantity of light to be received with respect to the thickness of intercalated tissue is examined in advance and the corresponding quantity of light to be emitted at that time is also set, and the thickness of intercalated tissue is calculated based on the quantity of light received when the set quantity of light is emitted onto the organism in actual measurement.

According to a further aspect of the present invention, the device for measuring the concentration of light absorbing material includes a single or a plurality of sensors having different distances between the sender and receiver of light; intercalated tissue thickness measuring means for measuring, using the one or more sensors, the thickness of intercalated tissue between the surface of an organism and the organic tissue in which the light absorbing material as a test target primarily exists; measurement sensitivity examining means for examining changes in measurement sensitivity with respect to the thickness of intercalated tissue at respective distances between the sender and receiver of light; coefficient of correction calculating means for determining, based on the change in measurement sensitivity, a relation between the thickness of intercalated tissue and the coefficient of correction for measurement sensitivity; and measured value correcting means for calculating, according to the above relation, the coefficient of correction from known distances of the sender and receiver of light of the one or more sensors and the thickness of intercalated tissue at a site of measurement, to correct the measured value based on the obtained coefficient of correction so as not to be influenced by the intercalated tissue.

More preferably, the intercalated tissue thickness measuring means is characterized in that it examines the quantity of light to be received with respect to the thickness of intercalated tissue in advance and also sets the corresponding quantity of light to be emitted at that time, and calculates the thickness of intercalated tissue based on the quantity of light actually received when the set quantity of light is emitted onto the organism upon measurement.

Preferably, the sensor is characterized in that it includes a light emitting element for emitting near infrared rays of a plurality of different wavelengths.

More preferably, the sensor is characterized in that the distance between the sender and receiver of light is set at 20mm, 30mm, or 40mm.

Brief Description of the Drawings

Fig. 1 is a block diagram showing a configuration of the measuring device according to a first embodiment of the present invention.

Figs. 2A to 2C are graphs showing changes in oxygen (oxygenated hemoglobin) when an exercise on a bicycle ergometer with and without load is repeated alternately.

Fig. 3 is a graph showing changes in oxygen (oxygenated hemoglobin) with respect to the thickness of adipose tissue at respective distances (20, 30, 40mm) between the sender and receiver of light.

Fig. 4 is a graph showing changes in light absorbency with respect to the thickness of adipose tissue at respective distances (20, 30, 40mm) between the sender and receiver of light.

Fig. 5 is a graph showing a relation between the thickness of adipose tissue and a coefficient of correction at respective distances (20, 30, 40mm) between the sender and receiver of light.

Fig. 6 is a graph showing changes in a current value in received light with respect to the thickness of adipose tissue at respective distances (20, 30, 40mm) between the sender and receiver of light.

Fig. 7 is a graph showing changes in intensity of received light with respect to the thickness of adipose tissue at respective distances (20, 30, 40mm) between the sender and receiver of light.

Fig. 8 is a graph showing a relation between the thickness of adipose tissue and the change in light absorbency, which illustrates a principle as for the measuring method according to a second embodiment of the present invention.

Figs. 9A and 9B are graphs showing relations between the quantity of emitted light and the quantity of received light, which illustrate the principle as for the measuring method according to the second embodiment of the present invention.

Fig. 10 is a graph showing a relation between the thickness of adipose tissue and logarithms of gradients with respective distances between the sender and receiver of light, which illustrates a variation of the measuring method according to the second embodiment of the present invention.

Figs. 11A to 11C show appearance of a configuration of a probe according to a third embodiment of the present invention.

Figs. 12A to 12C are views of a probe having a distance of 10mm between a light-emitting member 110 and a light-receiving member 112.

Figs. 13A to 13C are views of a probe having a distance of 20mm between light-emitting member 110 and light-receiving member 112.

Figs. 14A to 14C are views of a probe having a distance of 30mm between light-emitting member 110 and light-receiving member 112.

Figs. 15A to 15C are views of a probe having a distance of 40mm between light-emitting member 110 and light-receiving member 112.

Fig. 16 is a block diagram showing a configuration of the measuring device according to a fourth embodiment of the present invention.

Best Mode for Implementing the Present Invention

The present invention will now be described in more detail with reference to the attached drawings.

[First Embodiment]

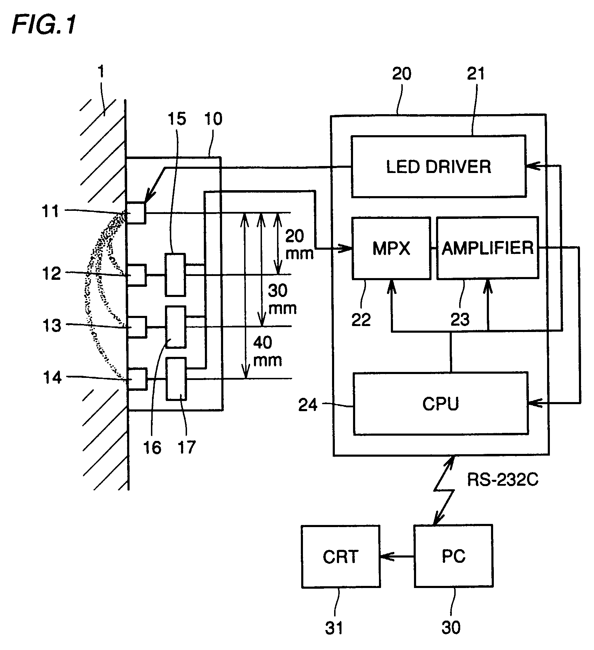

Fig. 1 is a block diagram showing a configuration of the measuring device according to the first embodiment of the present invention.

The measuring device is composed of a probe 10 mounted on the surface of an organism (tissue) 1, and a body 20 connected to probe 10 via a cable, for example.

Probe 10 includes a light-emitting element 11 as a light source for irradiating light onto organism 1, three photo diodes (PDs) 12, 13, 14 as light-receiving elements for receiving light reflected from organism 1, and first stage amplifiers 15, 16, 17 for amplifying received-light signals of PDs 12, 13, 14 to low impedance signals, respectively, to improve their signal to noise ratios.

Light-emitting element 11 in this embodiment consists of a light-emitting diode emitting near infrared rays with two wavelengths (LED, wavelengths: 760nm, 840nm), and PDs 12, 13, 14 are disposed at respective distances of 20mm, 30mm, and 40mm from LED 11. Accordingly, light reflected from body 1 can be received at three different locations at the same time. In this embodiment, LED 11 and PDs 12-14 constitute a sensor.

Body 20 includes an LED driver 21 for driving LED 11, a multiplexer 22 for switching signals from respective PDs 12, 13, 14, an amplifier 23 for amplifying the switched signal, and a CPU 24. Though not shown in Fig. 1, body 20 further includes an LCD, a memory card interface, a power supply, and so on. Amplifier 23 amplifies the signal amplified at the first stage by a factor of 20 to 2000 with gains in seven stages.

CPU 24 controls timing for light emission such that LED 11 lights up alternately at different wavelengths, and also adjusts the quantity of light by means of a D/A converter. CPU 24 has the following functions: an intercalated tissue thickness measuring function which uses a sensor to measure the thickness of intercalated tissue between the surface of the organism and organic tissue in which the light absorbing material to be tested primarily exists; a measurement sensitivity calculating function for examining changes in the measurement sensitivity with respect to the thickness of intercalated tissue at respective distances between the sender and receiver of light; a coefficient of correction calculating function for determining, based on the change in measurement sensitivity, a relation between the thickness of intercalated tissue and a coefficient of correction for measurement sensitivity; and a measured value correcting function for calculating, based on the relation obtained above, the coefficient of correction from known distances between the sender and receiver of light of the sensor and the thickness of intercalated tissue at the site of measurement, and correcting the measured value based on the obtained coefficient of correction so as not to be affected by the intercalated tissue. The LCD displays necessary information to confirm measurement states and conditions.

The measuring device formed of probe 10 and body 20 may be used independently (which is called an "off-line use"), or, as shown in Fig. 1, it can be used by connecting body 20 to a computer (personal computer) 30 via a serial input/output line such as RS-232C, and by further connecting CRT 31 to personal computer 30 (which is called an "on-line use").

In the on-line use, measured data of body 20 are taken into personal computer 30 via RS-232C, and are displayed in real time as changes in the concentration of light absorbing material (oxygenated hemoglobin or the like), changes in the quantity of blood, the thickness of intercalated tissue, and so on. In the off-line use, the data are stored in an inserted memory card.

A method of correcting a measured value using the measuring device configured as above will now be described.

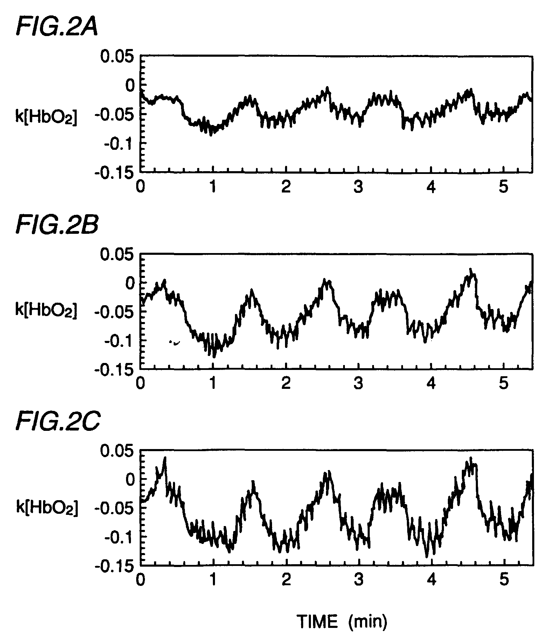

First, the test subject performs an exercise on a bicycle ergometer with a load of 120W and without load (idle pedaling) switched at every 30 seconds. The measuring device as shown in Fig. 1 is used to measure changes in the quantity of oxygen contained in the femoral region vastus lateralis muscle of the test subject during exercise. The results of measurement are shown in Figs. 2A to 2C.

Here, the concentration of oxygenated hemo-globin ($HbO_2$) within the organic tissue is measured. Fig 2A shows the result of measurement with a distance of 20 mm between the sender and receiver of light, Fig. 2B is with a distance of 30mm, and Fig. 2C is with a distance of 40mm. As seen in Figs. 2A to 2C, the measurement sensitivity of changes in oxygen increases as the distance between the sender and receiver of light increases. This occurs because the average length of the paths of light becomes longer as the distance between the sender and receiver of light increases, thereby permitting measurement of deeper tissue from the surface.

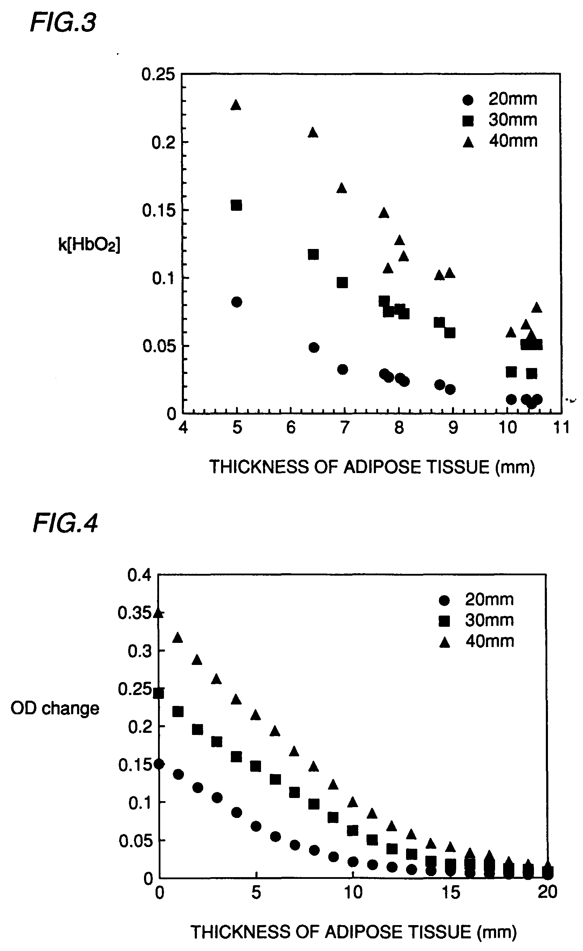

Measurement was performed on 13 test subjects, and according to the measured values, average values of variation in the concentration of oxygen with respective distances between the sender and receiver of light were plotted on Fig. 3 (a relation between the thickness of adipose tissue and the concentration of oxygen). Results of theoretical calculation by the Monte Carlo simulation method are shown in Fig. 4 (a relation between the thickness of adipose tissue and the change in light absorbency). The results of each correspond well, thereby showing that measurement sensitivity varies according to the thickness of adipose tissue, i.e., measurement sensitivity considerably decreases as the adipose tissue becomes thicker, regardless of the distance between the sender and receiver of light.

Procedures for correcting measurement sensitivity are as follows. As shown in Fig. 4, for example, theoretical curves representing changes in light absorbency (measurement sensitivity) with respect to the thickness of adipose tissue at respective distances between the sender and receiver of light are used to obtain an approximation polynomial expressed therein. From this approximation polynomial, a rate of change in oxygen, when the thickness of adipose tissue is 0, with a respective distance between the sender and receiver of light is calculated, and the inverse of the obtained rate is set as a coefficient of correction.

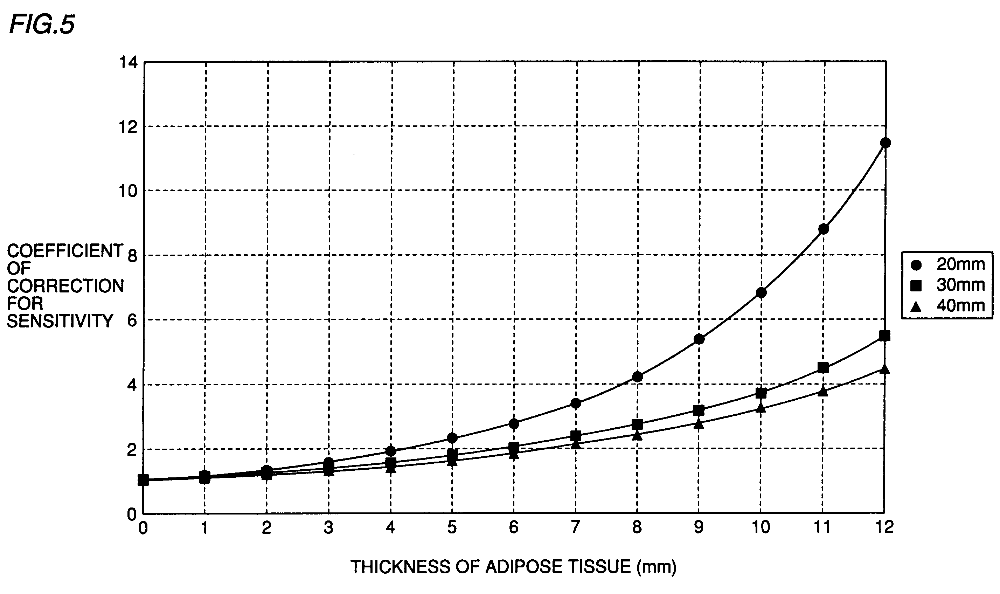

Fig. 5 is a graph showing the coefficient of correction compared to the thickness of adipose tissue with a respective distance between the sender and receiver of light, in a practical range of the thickness of adipose tissue (in the order of 0-12 mm).

In this relation between the thickness of adipose tissue and the coefficient of correction, if the distance between the sender and receiver of light and the thickness of adipose tissue at the site of measurement are known, the coefficient of correction at that time can be obtained. Further, by multiplying a measured value by the coefficient of correction, the measured value can be corrected to a measurement sensitivity (i.e. a measured value) where the thickness of adipose tissue being 0. For example, if the distance between the sender and receiver of light is 20mm and the thickness of adipose tissue is 8mm, the coefficient of correction is approximately 4.2 as shown in Fig. 5. By multiplying the measured value by 4.2, a measured value uninfluenced by the thickness of adipose tissue can thus be obtained.

Conversely, referring to Fig. 3 which shows the relation between the thickness of adipose tissue and measurement sensitivity, if the thickness of adipose tissue and the distance between the sender and receiver of light are known, measurement sensitivity at that time can be corrected.

In the above embodiment, a known thickness of adipose tissue has been used to obtain a coefficient of correction. However, if the thickness of adipose tissue is to be measured using the measuring device, the following procedures, for example, can be used.

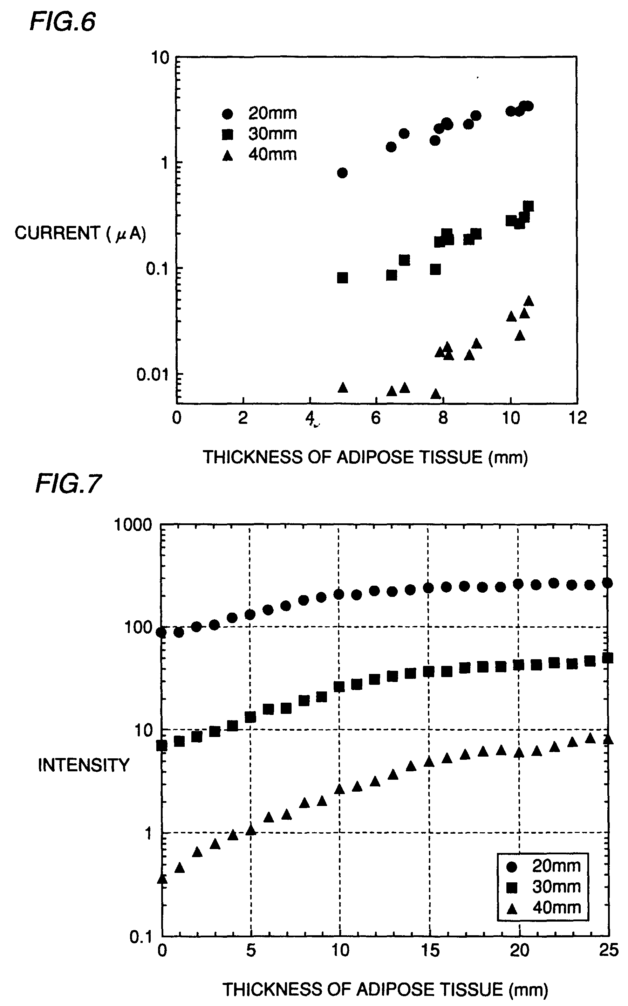

Fig. 6 is a graph showing a relation between the thickness of adipose tissue and the distance between the sender and receiver of light, when an initial value of light measured with probe 11 attached to the area to be measured is expressed as an electric current value of light received at the respective light-receiving elements 12, 13, and 14.

The result of theoretical calculation regarding the same relation (relation between the thickness of adipose tissue and the intensity of received light at a respective distance between the sender and receiver of light) according to the Monte Carlo simulation method is shown in Fig. 7. The figures show that nearly the same results were obtained in each.

Accordingly, the quantity of light to be received with respect to the thickness of adipose tissue in all cases is calculated in advance and the corresponding quantity of light to be emitted is also set. In actual measurement, the quantity of light emitted is calibrated to the set quantity by means of a phantom or the like. From the quantity of light received when that quantity of light is emitted onto the organism, the thickness of adipose tissue at the measured site can be obtained using the following calculation taken from the theoretical calculation in Fig. 7. The thickness of adipose tissue H is expressed as follows:

$$H=\{\log(I/2200)+1.241\}/0.113$$

(I: 0 to 3600 on A/D input with an emitted light wavelength of 840nm with a distance of 40mm between the sender and receiver of light)

Though the thickness of adipose tissue has been measured by the measuring device by way of example in this embodiment, it may be measured by other measuring methods such as ultrasound technology. The thickness of adipose tissue has been measured in this embodiment; however, other kinds of intercalated tissue (skull, skin, cerebrospinal fluid and so on) can be measured likewise, allowing measurement of the concentration of light absorbing material without being influenced by their thicknesses.

Further, in this embodiment, one LED and three PDs have been used as a sensor. Though three LEDs and one PD can be used conversely, or one LED and one PD can be used, a plurality of sensors having different distances between the sender and receiver of light can broaden a region for measurement.

Instead of measuring the thickness of adipose tissue of an organism as in this embodiment, the thickness of material other than an organism like meat, for example, can be measured likewise.

As described above, the method and apparatus for measuring the concentration of light absorbing material within organic tissue according to this embodiment enable measurement of the concentration of light absorbing material and eliminates the influence of the thickness of intercalated tissue (skin, skull, subcutaneous adipose tissue, cerebrospinal fluid and so on) between the surface of the organism and the organic tissue of the test subject, thereby bringing about the following effects:

(1) Difference in measurement among individuals can be eliminated, allowing quantitative comparison of measured values among individuals.
(2) The measured value can be made an absolute value indicator.
(3) Information about the thickness of intercalated tissue can be displayed at the time of measurement.
(4) With the effects (1) through (3), the measured value has physiological significance, and is quite useful for clinical diagnosis.

[Second Embodiment]

The hardware configuration of the measuring device according to the second embodiment of the present invention is identical to that of the first embodiment, and therefore, description thereof is not repeated.

Now, a principle as for measuring the thickness of intercalated tissue (adipose tissue) within an organism by the measuring device according to this embodiment will be described.

(First Measuring Method)

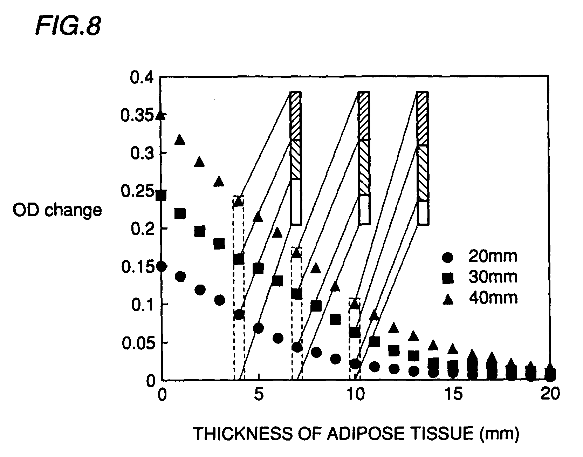

Initially, the first measuring method will be described. Fig. 8 is a graph showing the relation between the thickness of adipose tissue obtained by PDs 12, 13, 14 arranged at different distances from LED 11 and the changes in light absorbency measured at the surface of organism when the light absorbency of muscular tissue changes.

As seen from this graph, the measurement sensitivity varies dependent on the thickness of adipose tissue, the degree of decrease in measurement sensitivity differs depending on the distance between the sender and receiver of light, and the rate of change in light absorbency varies dependent on the distances. Here, the rates of changes in light absorbency at respective distances when the thickness of adipose tissue is about 4mm, 7mm, and 10mm are shown in the bar graphs in Fig. 8. According to the rates of changes in light absorbency, the change in light absorbency with a distance of 20mm between the sender and receiver of light is relatively large when the adipose tissue is thin, while the changes in light absorbency with distances of 30mm, 40mm between the sender and receiver of light are relatively large when the adipose tissue is thick.

Accordingly, in the actual range of measurement (with the thickness of adipose tissue on the order of 4-12 mm), the thickness of adipose tissue can be calculated by obtaining the rate of change in light absorbency with respective distances between the sender and receiver of light.

It is possible to calculate the thickness of adipose tissue by using the fluctuation itself as it occurs during the measurement of the thickness of adipose tissue. However, if there is little fluctuation in measurement, or when the thickness of adipose tissue is to be calculated before measurement by this method, it is necessary to alter the oxygen concentration. To achieve this, the following procedures, for example, may be taken:

- Press the probe hard to the area to be tested of the organism.
- Suppress blood flow to the measurement area by means of a cuff.
- Heat the area to be measured.
- Place the site to be measured higher or lower with respect to the height of the heart.

(Second Measuring Method)

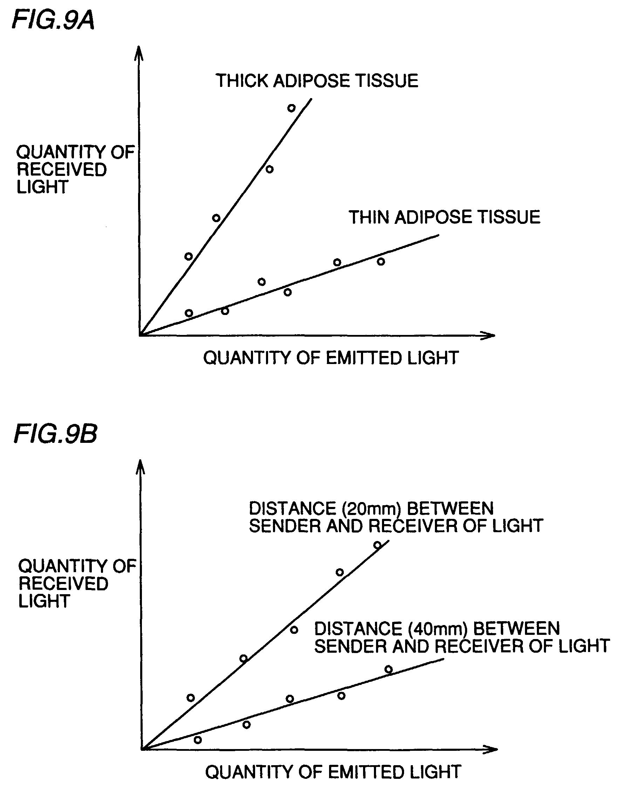

The second measuring method will now be described. When the quantity of light emitted from LED 11 is altered linearly, the quantity of light received with respect to the thickness of adipose tissue changes linearly as shown in Fig. 9A. The graph shows that the change in the quantity of light received is larger when the adipose tissue is thicker. Likewise, the quantity of light received with respect to the distance between the sender and receiver of light when the quantity of light emitted from LED 11 is altered linearly also changes linearly as shown in Fig. 9B. According to the graph, the change in the quantity of light received is smaller when the distance between the sender and receiver of light is larger. In other words, a coefficient of proportionality according to the linear change is large when the adipose tissue is thick, while the coefficient of proportionality according to the linear change is small when the distance between the sender and receiver of light is far.

Accordingly, it becomes possible to obtain the coefficient of proportionality for the linear change from the known distance between the sender and receiver of light (here, 20mm, 30mm, 40mm), and to calculate the thickness of adipose tissue based on the obtained coefficient of proportionality.

As a variation to this measuring method, it is also possible to calculate the thickness of adipose tissue by obtaining the coefficients at respective, different distances between the sender and receiver of light, and calculating the rates of those coefficients.

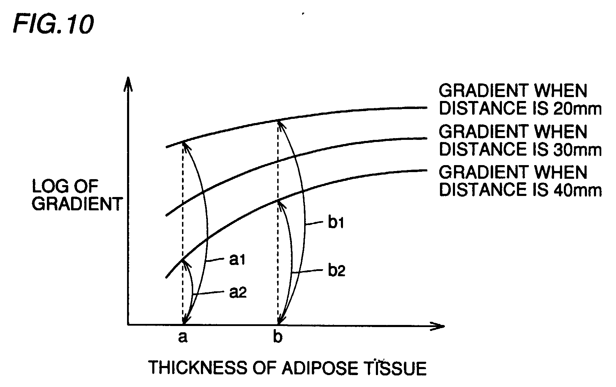

Here, the distances from the sender to receiver of light are assumed to be, for example, 20mm, 30mm, 40mm. When plotting logarithms of gradients at respective distances as shown in Fig. 10, the rate of coefficients at distances 20mm and 40mm for the thickness a and b of adipose tissue becomes a1/a2, b1/b2, respectively. This means that the rate of coefficients differs depending on the thickness of adipose tissue. Therefore, obtaining a rate of coefficients with different distances between the sender and receiver of light enables calculation of the thickness of adipose tissue.

The second measuring method is advantageous in that there is no need to first calibrate the quantity of light emitted from LED 11. Furthermore, since the thickness of adipose tissue is calculated from a plurality of data, stable and precise calculation of the thickness of adipose tissue is ensured. Saturation of the quantity of light is not likely, the range of calculable thickness of adipose tissue is broadened, and optimum distance between the sender and receiver of light as well as optimum quantity of light for measurement can advantageously be selected.

In the above embodiment, the procedure for measuring the thickness of adipose tissue as intercalated tissue has been described. However, this also applies to the case of measurement of brain tissue, in which skull, cerebrospinal fluid, and others are to be measured as intercalated tissue between the surface of the head and the brain tissue.

In addition, though one LED and three PDs have been used as a sensor in this embodiment, three LEDs and one PD can be used instead, or, one LED and one PD can be used. However, using a plurality of sensors with different distances from the sender to receivers of light can broaden a measurable region.

Furthermore, the present embodiment has been used for measurement of an organism. However, this can also be used for measurement of meat or the like, instead of the organisms.

As described above, the method and apparatus for measuring the thickness of intercalated tissue within an organism according to the present embodiment allow measurement of the thickness of intercalated tissue (skin, skull, subcutaneous adipose tissue, cerebrospinal fluid, and so on) between the organic tissue which is expected to be actually measured and the surface of the organism. Accordingly, not only the thickness of intercalated tissue can be measured, but also using this, precise measurement of oxygen in the organic tissue as a test target is guaranteed without being affected by the thickness of intercalated tissue.

[Third Embodiment]

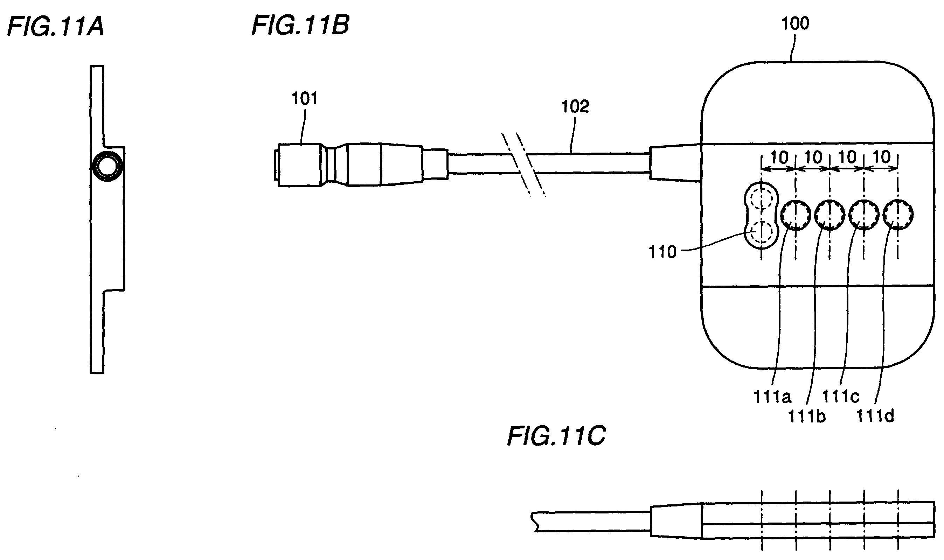

Figs. 11A to 11C show a probe used with the measuring device according to the third embodiment of the present invention. Specifically, Fig. 11A shows its side view, Fig. 11B shows its plan view, and Fig. 11C shows its front view.

Referring to the drawings, the prove is composed of a connector 101 for connection with the body, a probe body 100 formed of silicon rubber for detachably attaching a light-receiving member, a cable 102 for connecting the connector with the probe body, a light-emitting member 110 secured at probe body 100, and light-receiving member attaching portions 111a-111d for detachably attaching the light-receiving member at distances from 10mm to 40mm from the light-emitting member, each spaced apart by 10mm from one another.

In this embodiment, light-emitting member 110 has two identical LEDs attached to increase the quantity of light. However, only one LED can be attached to the probe body if it can emit a sufficient amount of light.

In this embodiment, the light-receiving member can detachably be placed at distances from 10mm to 40mm from the light-emitting member, with spaces of 10mm each therebetween. Accordingly, without having to use a plurality of light-receiving members, measurement can be conducted two to four times by disposing the light-receiving member at different positions, and by combining the results. The effects similar to those in the first and second embodiments can thus be obtained.

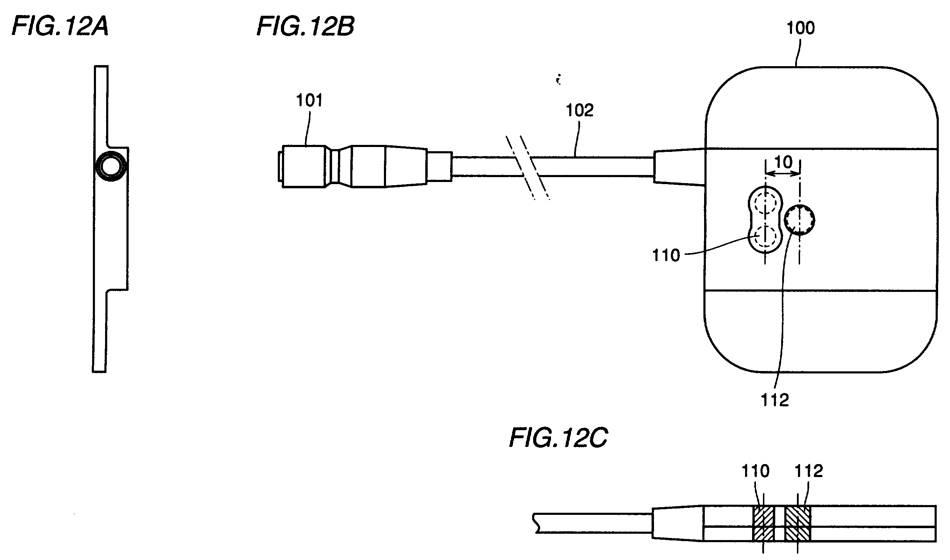

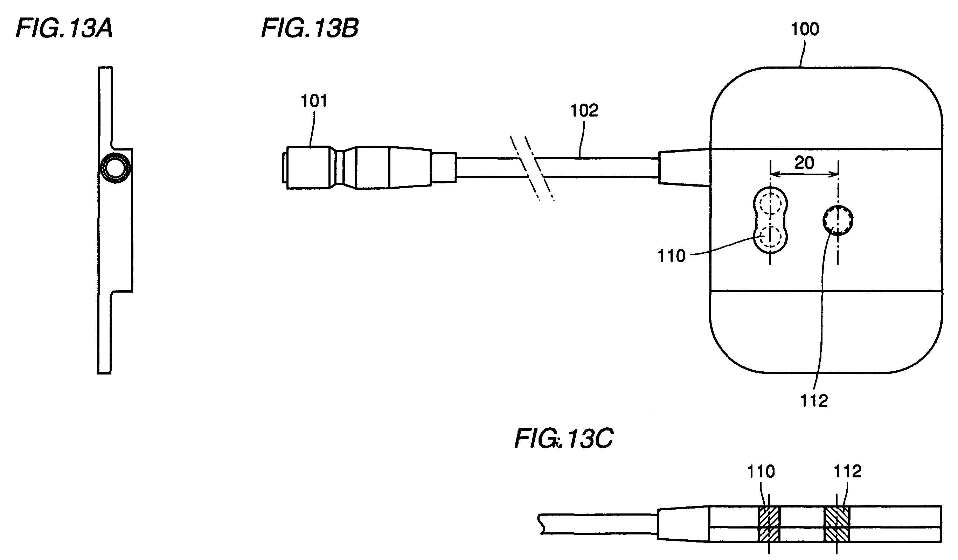

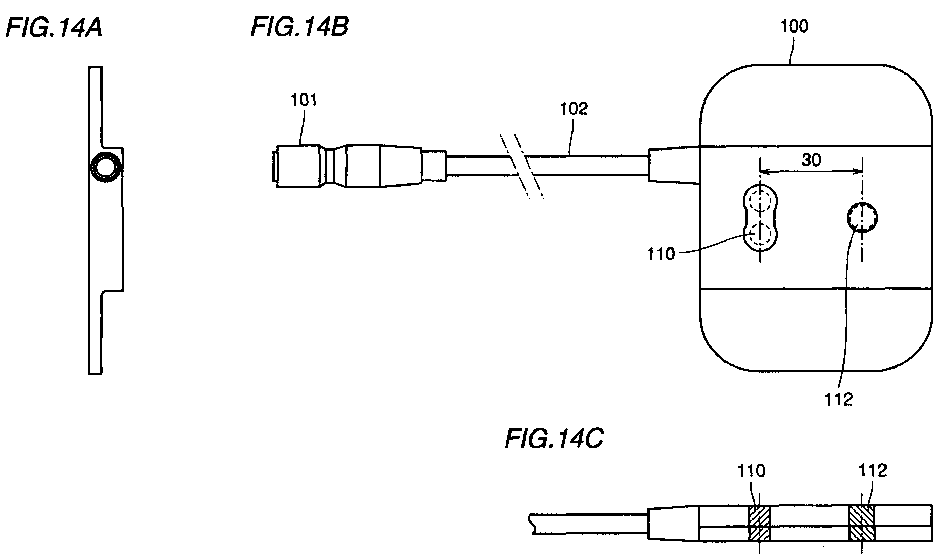

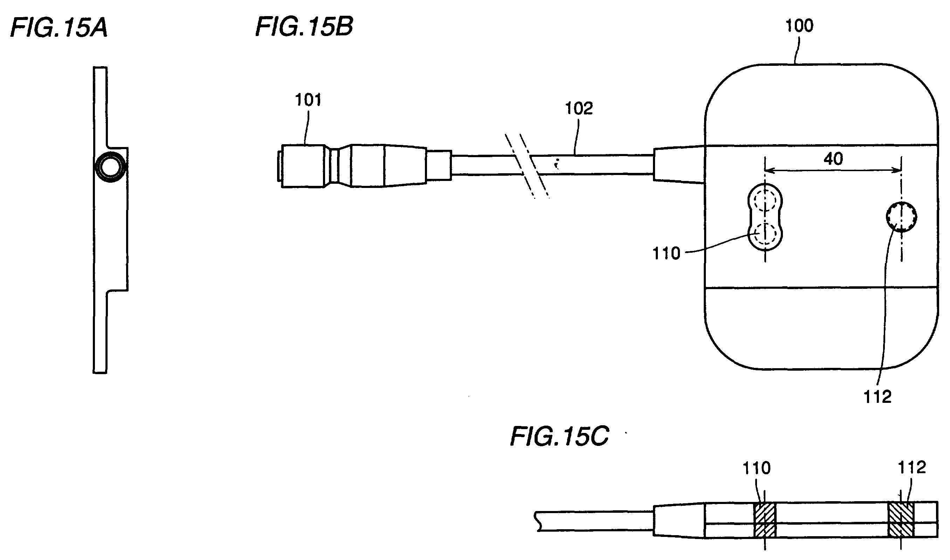

Figs. 12A through 15C are graphs each showing the case in which the distance between the light-emitting member and the light-receiving member is 10mm, 20mm, 30mm, and 40mm, respectively.

[Fourth Embodiment]

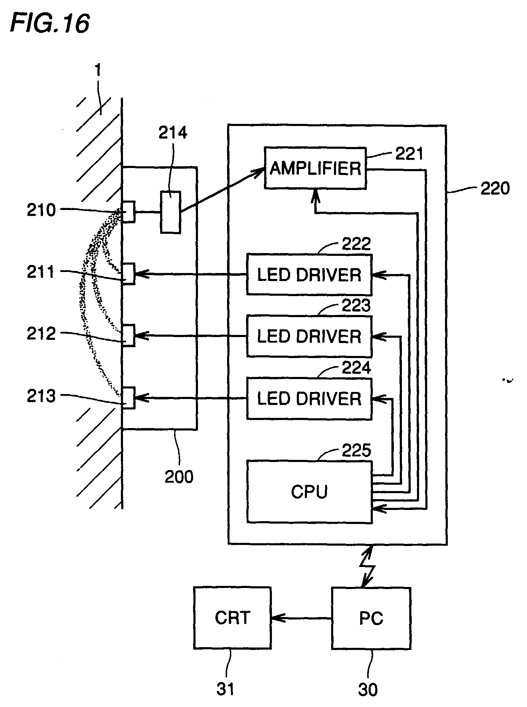

Fig. 16 is a block diagram showing a configuration of the measuring device according to the fourth embodiment of the present invention.

Referring to the drawing, the measuring device is generally composed of a body 220 of the measuring device and a probe 200.

The probe includes light-emitting diodes (LEDs) 211-213, a photo diode (light-receiving element) 210, and a first stage amplifier 214 for amplifying a signal from the photo diode to a low impedance signal. In measurement, probe 200 is attached to organic tissue 1.

Body 220 includes am amplifier 221 further amplifying the signal from amplifier 214, LED drivers 222-224 for driving the light-emitting diodes, and a CPU 225.

Body 220 is connected to a personal computer 30 or a CRT31.

In the present embodiment, one photo diode 210 and three light-emitting diodes 211-213 are used as a sensor. Accordingly, the distance between the photo diode and the light-emitting diode can be altered as in the first and second embodiments, for measuring the concentration of light absorbing material within the organic tissue or the thickness of intercalated tissue.

Applicability to the Industrial Field

As described above, the present invention permits accurate measurement of the concentration of light absorbing material within organic tissue as well as the thickness of intercalated tissue of an organism. Accordingly, the present invention is advantageously applicable to the fields of medical equipment and so on.

**Claims**

1.  A method of measuring the thickness of light absorbing material, comprising the steps of measuring changes in light absorbency at the surface of a material by a plurality of sensors having different distances between sender and receiver of light thereof, and calculating the thickness of light absorbing material based on the rate of the change obtained in light absorbency.

2.  A method of measuring the thickness of light absorbing material within an organism, comprising the steps of measuring changes in light absorbency within organic tissue by one or a plurality of sensors having different distances between sender and receiver of light thereof, and calculating the thickness of light absorbing material based on the rate of the change obtained in light absorbency.

3.  A method of measuring the thickness of adipose tissue within an organism, comprising the steps of measuring changes in light absorbency at the surface of the organism by one or a plurality of sensors having different distances between sender and receiver of light thereof, and calculating the thickness of adipose tissue within the organism based on the rate of the change obtained in light absorbency.

4.  A method of measuring the thickness of light absorbing material of an organism, comprising the steps of measuring, by one or a plurality of sensors having different distances between sender and receiver of light thereof, a linear change in the quantity of light received when the quantity of light emitted onto the organism is altered linearly; obtaining a coefficient of proportionality in the obtained linear change from known distances of sender and receiver of light of the one or plurality of sensors; and calculating the thickness of light absorbing material based on the obtained coefficient of proportionality.

**5.** A device for measuring thickness of a light absorbing material, comprising:

one or a plurality of sensors having different distances between sender and receiver of light thereof;

light absorbency change measuring means for measuring, using the one or plurality of sensors, changes in light absorbency with respect to the thickness of light absorbing material; and

light absorbing material thickness calculating means for calculating the thickness of light absorbing material based on the rate of the change obtained in light absorbency.

**6.** A device for measuring thickness of adipose tissue of an organism, comprising:

one or a plurality of sensors having different distances between sender and receiver of light thereof;

light absorbency change measuring means for measuring, using the one or plurality of sensors, changes in light absorbency at the surface of the organism; and

adipose tissue thickness calculating means for measuring the thickness of adipose tissue based on the rate of the change obtained in light absorbency.

**7.** A device for measuring thickness of intercalated tissue of an organism, comprising:

one or a plurality of sensors having different distances between the sender and receiver of light thereof,

quantity of received light measuring means for measuring a linear change in the quantity of light received when the quantity of light emitted onto the organism is altered linearly; and

intercalated tissue thickness calculating means for determining a coefficient of proportionality in the obtained linear change from known distances of sender and receiver of light of the one or plurality of sensors, and calculating, based on the determined coefficient of proportionality, the thickness of intercalated tissue between the surface of the organism and organic tissue in which a light absorbing material as a test target primarily exists.

**8.** The device for measuring the thickness according to one of claims 5 to 7, wherein said sensor includes an element for emitting light with a plurality of different wavelengths.

**9.** The device for measuring the thickness according to one of claims 5 to 7, comprising a plurality of mounting portions each for detachably securing a light-emitting element or a light-receiving element of said sensor, wherein

a probe is provided with different distances between the sender and receiver of light altered by means of the plurality of mounting portions.

**10.** A method of measuring concentration of a light absorbing material within organic tissue, comprising the steps of:

measuring, by one or a plurality of sensors having different distances between sender and receiver of light thereof, the thickness of intercalated tissue between the surface of the organism and organic tissue in which the light absorbing material as a test target primarily exists;

examining changes in measurement sensitivity with respect to the thickness of intercalated tissue at respective distances between the sender and receiver of light;

determining, based on the change in measurement sensitivity, a relation between the thickness of intercalated tissue and a coefficient of correction for measurement sensitivity; and

calculating, according to the relation, the coefficient of correction from known distances of the sender and receiver of light of said one or plurality of sensors and the thickness of intercalated tissue at a site to be measured, and correcting a measured value so as not to be influenced by the intercalated tissue.

**11.** The method of measuring the concentration of the light absorbing material within the organic tissue according to claim 10, wherein the quantity of light to be received with respect to the thickness of intercalated tissue is determined in advance and the quantity of light to be emitted at that time is also set, and the thickness of said intercalated tissue is calculated based on the quantity of light received when the set quantity of light is emitted onto the organism in actual measurement.

**12.** A device for measuring concentration of a light absorbing material within organic tissue, comprising:

one or a plurality of sensors having different distances between sender and receiver of light thereof;

intercalated tissue thickness measuring means for measuring, by the one or plurality of sensors, the thickness of intercalated tissue between the surface of the organism and the organic tissue in which the light absorbing material as a test target primarily exists;

measurement sensitivity calculating means for determining changes in the measurement sensitivity with respect to the thickness of intercalated tissue at respective distances between the sender and receiver of light;

coefficient of correction measuring means for determining, based on the change in the measurement sensitivity, a relation between the thickness of intercalated tissue and a coefficient of correction for measurement sensitivity; and

measured value correcting means for calculating, according to the relation, the coefficient of correction from known distances between the sender and receiver of light of said one or plurality of sensors and the thickness of intercalated tissue at the site of measurement, and correcting, based on the obtained coefficient of correction, a measured value so as not to be influenced by the intercalated tissue.

13. The device for measuring the concentration of the light absorbing material within the organic tissue according to claim 12, wherein said intercalated tissue thickness measuring means determines the quantity of light to be received with respect to the thickness of intercalated tissue in advance and also sets the quantity of light to be emitted at that time, and performs the calculation based on the quantity of light received when the set quantity of light is emitted onto the organism in actual measurement.

14. The device for measuring the concentration of the light absorbing material within the organic tissue according to claim 12 or 13, wherein each of said one or plurality of sensors has an element emitting near infrared rays with a plurality of different wavelengths.

15. The device for measuring the concentration of the light absorbing material within the organic tissue according to claim 12, wherein the distance between the sender and receiver of light of said one or plurality of sensors is set at 20mm, 30mm, or 40mm.

16. The device for measuring the concentration of the light absorbing material within the organic tissue according to claim 13, wherein the distance between the sender and receiver of light of said one or plurality of sensors is set at 20mm, 30mm, or 40mm.

17. The device for measuring the concentration of the light absorbing material within the organic tissue according to claim 14, wherein the distance between the sender and receiver of light of said one or plurality of sensors is set at 20mm, 30mm, or 40mm.

## FIG.1

*FIG.2A*

*FIG.2B*

*FIG.2C*

TIME (min)

*FIG.3*

THICKNESS OF ADIPOSE TISSUE (mm)

*FIG.4*

THICKNESS OF ADIPOSE TISSUE (mm)

*FIG.5*

COEFFICIENT OF CORRECTION FOR SENSITIVITY

THICKNESS OF ADIPOSE TISSUE (mm)

- ● 20mm
- ■ 30mm
- ▲ 40mm

EP 0 942 260 A1

*FIG.6*

Legend:
- ● 20mm
- ■ 30mm
- ▲ 40mm

Y-axis: CURRENT ($\mu$A)
X-axis: THICKNESS OF ADIPOSE TISSUE (mm)

*FIG.7*

Y-axis: INTENSITY
X-axis: THICKNESS OF ADIPOSE TISSUE (mm)

Legend:
- ● 20mm
- ■ 30mm
- ▲ 40mm

## FIG.8

## FIG.9A

QUANTITY OF RECEIVED LIGHT

THICK ADIPOSE TISSUE

THIN ADIPOSE TISSUE

QUANTITY OF EMITTED LIGHT

## FIG.9B

QUANTITY OF RECEIVED LIGHT

DISTANCE (20mm) BETWEEN SENDER AND RECEIVER OF LIGHT

DISTANCE (40mm) BETWEEN SENDER AND RECEIVER OF LIGHT

QUANTITY OF EMITTED LIGHT

## FIG.10

LOG OF GRADIENT

GRADIENT WHEN DISTANCE IS 20mm

GRADIENT WHEN DISTANCE IS 30mm

GRADIENT WHEN DISTANCE IS 40mm

b1

a1

b2

a2

a    b

THICKNESS OF ADIPOSE TISSUE

*FIG.11A*

*FIG.11B*

100

101

102

10 | 10 | 10 | 10

110

111a    111c
    111b    111d

*FIG.11C*

EP 0 942 260 A1

FIG.12A

FIG.12B

FIG.12C

EP 0 942 260 A1

EP 0 942 260 A1

*FIG.13A*

*FIG.13B*

100

101

102

20

110

112

*FIG.13C*

110   112

FIG.14A

FIG.14B

FIG.14C

EP 0 942 260 A1

*FIG.15A*

*FIG.15B*

100

101

102

40

110

112

EP 0 942 260 A1

*FIG.15C*

110

112

*FIG.16*

# INTERNATIONAL SEARCH REPORT

| International application No. |
| --- |
| PCT/JP97/04301 |

**A. CLASSIFICATION OF SUBJECT MATTER**
Int.Cl$^6$ G01B11/06, A61B5/14, 310

According to International Patent Classification (IPC) or to both national classification and IPC

**B. FIELDS SEARCHED**

Minimum documentation searched (classification system followed by classification symbols)
Int.Cl$^6$ G01B11/00-30, A61B5/06-22

Documentation searched other than minimum documentation to the extent that such documents are included in the fields searched

| | | | |
| --- | --- | --- | --- |
| Jitsuyo Shinan Koho | 1926-1996 | Toroku Jitsuyo Shinan Koho | 1994-1998 |
| Kokai Jitsuyo Shinan Koho | 1971-1998 | Jitsuyo Shinan Toroku Koho | 1996-1998 |

Electronic data base consulted during the international search (name of data base and, where practicable, search terms used)

**C. DOCUMENTS CONSIDERED TO BE RELEVANT**

| Category* | Citation of document, with indication, where appropriate, of the relevant passages | Relevant to claim No. |
| --- | --- | --- |
| P,A | JP, 9-168531, A (Johnson & Johnson Medical Inc.), June 30, 1997 (30. 06. 97) & EP, 760476, A | 1-17 |
| A | JP, 8-182654, A (Kao Corp.), July 16, 1996 (16. 07. 96) | 1-17 |
| A | JP, 4-132540, A (Kao Corp.), May 6, 1992 (06. 05. 92) | 1-17 |

☐ Further documents are listed in the continuation of Box C.   ☐ See patent family annex.

| | | | |
| --- | --- | --- | --- |
| * | Special categories of cited documents: | "T" | later document published after the international filing date or priority date and not in conflict with the application but cited to understand the principle or theory underlying the invention |
| "A" | document defining the general state of the art which is not considered to be of particular relevance | | |
| "E" | earlier document but published on or after the international filing date | "X" | document of particular relevance; the claimed invention cannot be considered novel or cannot be considered to involve an inventive step when the document is taken alone |
| "L" | document which may throw doubts on priority claim(s) or which is cited to establish the publication date of another citation or other special reason (as specified) | | |
| "O" | document referring to an oral disclosure, use, exhibition or other means | "Y" | document of particular relevance; the claimed invention cannot be considered to involve an inventive step when the document is combined with one or more other such documents, such combination being obvious to a person skilled in the art |
| "P" | document published prior to the international filing date but later than the priority date claimed | "&" | document member of the same patent family |

| Date of the actual completion of the international search | Date of mailing of the international search report |
| --- | --- |
| February 17, 1998 (17. 02. 98) | March 3, 1998 (03. 03. 98) |

| Name and mailing address of the ISA/ | Authorized officer |
| --- | --- |
| Japanese Patent Office | |
| Facsimile No. | Telephone No. |

Form PCT/ISA/210 (second sheet) (July 1992)